(19)

Europäisches
Patentamt

European
Patent Office

Office européen
des brevets

(11) **EP 3 868 894 A1**

(12) **EUROPEAN PATENT APPLICATION**

(43) Date of publication:
**25.08.2021 Bulletin 2021/34**

(51) Int Cl.:
**C12Q 1/6883** [(2018.01)]

(21) Application number: **20158652.6**

(22) Date of filing: **21.02.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Forschungszentrum Borstel, Leibniz Lungenzentrum**
**23845 Borstel (DE)**

(72) Inventors:
• **HEYCKENDORF, Jan**
**20359 Hamburg (DE)**

• **MARWITZ, Sebastian**
**23858 Reinfeld (DE)**
• **REIMANN, Maja**
**22359 Hamburg (DE)**
• **GOLDMANN, Torsten**
**23858 Reinfeld (DE)**
• **LANGE, Christoph**
**23843 Bad Oldesloe (DE)**

(74) Representative: **Gramm, Lins & Partner Patent- und Rechtsanwälte PartGmbB**
**Freundallee 13a**
**30173 Hannover (DE)**

(54) **METHOD FOR DIAGNOSIS AND TREATMENT MONITORING AND INDIVIDUAL THERAPY END DECISION IN TUBERCULOSIS INFECTION**

(57)     The present invention relates in a first aspect to a method for diagnosing tuberculosis infection calculating a score based on the expression of a predetermined set of genes and as well as diagnosing or predicting the clinical outcome of the tuberculosis infection based on said score. In another aspect, a method for treatment monitoring and determining treatment duration in an individual with tuberculosis infection is provided. Said method includes the determination of the expression level of predetermined genes, calculating a value based thereon and the therapy monitoring or treatment monitoring and determining treatment duration in said individual accordingly. Moreover a method for determining the therapy-end of a treatment of tuberculosis infection in an individual affected with tuberculosis infection is provided.

Said method includes the consideration of the expression level of predetermined genes together with the scores and values identified and calculating whether therapy is completed or not based on the value, the score and the expression level of said predetermined genes accordingly. In a further aspect, the use of a test kit or the use of kits of parts for conducting the methods according to the present invention is provided. Further, an array comprising means for detecting the expression of predetermined genes is identified as well as the use of the same. Moreover, a computer implemented method is provided as well as a computer readable medium or computer program product having computer executable instructions for performing the steps of the method disclosed herein.

EP 3 868 894 A1

**Description**

The present invention relates in a first aspect to a method for diagnosing tuberculosis infection calculating a score based on the expression of a predetermined set of genes as well as diagnosing or predicting the clinical outcome of the tuberculosis infection based on said score. In another aspect, methods for therapy monitoring or treatment monitoring and determining treatment duration in an individual with tuberculosis infection are provided. Said methods include the determination of the expression level of predetermined genes, calculating a value based thereon and allowing treatment monitoring and determining the treatment duration in said individual accordingly. Moreover, a method for determining the therapy-end of a treatment of tuberculosis infection in an individual afflicted with tuberculosis infection is provided. Said method includes the consideration of the expression level of predetermined genes together with the scores and value determined according to the present invention and calculating whether therapy is completed or not based on the value, the score and the expression level of said predetermined genes accordingly. In a further aspect, the use of a test kit or the use of a kit of parts for conducting the methods according to the present invention is provided. Further, an array comprising means for detecting the expression of predetermined genes is identified as well as the use of the same. Moreover, a computer-implemented method is provided as well as a computer readable medium or computer program product having computer executable instructions for performing the steps of the method disclosed herein.

Prior art

Tuberculosis (TB) remains a major global health threat with emerging *Mycobacterium tuberculosis* drug-resistance being particularly worrisome. Multidrug-resistant TB (MDR-TB), defined by bacillary resistance against rifampicin and isoniazid, and extensively drug-resistant TB (XDR-TB), defined by MDR-TB plus resistance against at least a fluoroquinolone and one of the second-line injectable drugs amikacin, cap-reomycin and/or kanamycin, are associated with very high treatment costs, frequently occurring severe adverse events, and discouragingly poor outcomes despite recommended treatment durations of 20 months with combination anti-TB therapies. The WHO has recently endorsed a shorter course MDR-TB treatment regimen with a duration of 9 - 12 months for patients with fluoroquinolone-susceptible TB who fulfill also other requirements. Unfortunately, however, the great majority of patients in some regions of the world, e. g. Europe, are not eligible for the short course regimen due to advanced levels of second-line *M. tuberculosis* drug-resistance. The term MDR-TB is used herein in the following and includes the form of MDR-TB and XDR-TB unless otherwise indicated.

It is assumed that individual duration of anti-TB drug-therapy required to achieve relapse-free cure is highly variable and depends on the host's immune status, the extent of the disease, the pathogen's virulence, drug-resistance and the active drugs available for the treatment, thus summarizing the most prominent factors. Accordingly, there is a growing interest in biomarkers that identify the optimal time point for the end of treatment in order to individualize the duration of anti-TB therapy, which is especially relevant for the treatment of patients with drug-resistant TB. Namely, in particular with respect to MDR-TB it would be very helpful to determine the therapy end.

Due to rapid changes in expression profiles following the initiation of anti-TB therapies, host ribonuclear acids (RNA) appear to be promising candidates to be evaluated as markers for an individual duration of anti-TB therapy needed to achieve relapse-free cure. Actually, RNA expression levels that correlate with treatment responses, predict patient outcome, and recurrence of disease have been previously described in individuals with non-MDR-TB (Darboe F., et al., Front Microbiol, 2019, 10, 1441; Thompson, E. G., et al., Tuberculosis (Edinb), 2017, 107, 48 - 58).

However, methods for determining the duration of therapy and the therapy regimen as well as for determining the treatment course in particular with respect to resistant tuberculosis have not been described so far.

Accordingly, there is a need for providing new methods for diagnosing or predicting the clinical outcome of tuberculosis infection in individuals afflicted with or suspected to be afflicted with tuberculosis infection as well as for methods for therapy monitoring or treatment monitoring and determining treatment duration of an individual afflicted with tuberculosis infection. Further, there is a need for a method for determining the therapy-end of a treatment of tuberculosis infection in an individual afflicted with tuberculosis infection accordingly. The corresponding need is particular high, when it comes to MDR-TB.

Brief description of the present invention

In a first aspect, the present invention relates to a method for diagnosing or predicting the clinical outcome of tuberculosis infection in an individual afflicted with or suspected to be afflicted with a tuberculosis infection, comprising

a) determining the expression level of the genes: CD274, FAM20A, GYG1, HIST1H1B, LPCAT2 and TRIM27 in a sample obtained from said individual;
b) calculating a score based on the expression level of said six genes identified above;

c) diagnosing or predicting the clinical outcome of tuberculosis infection based on the score calculated in step b).

In another aspect, the present invention relates to a method for treatment monitoring and determining treatment duration in an individual with tuberculosis infection, comprising the steps of:

a) determining the expression level of the genes: RPAP3, A_33_P3281041, BATF2, C2, GK3P, IFIT2, IFITM1, KREMEN1 and PDE4D in a sample from said individual;
b) calculating a value based on the expression level of said genes identified in step a);
c) monitoring treatment or determining treatment duration in said individual based on the value calculated in step b).

Moreover, the present invention provides a method for determining the therapy-end of a treatment of tuberculosis infection in an individual afflicted with tuberculosis infection, comprising:

a) determining in a sample of said individual the expression level of the genes CD274, FAM20A, GYG1, HIST1H1B, LPCAT2 and TRIM27, preferably, calculating the score according to the method described above;
b) determining in a sample of said individual the expression level of the genes RPAP3, A_33_P3281041, BATF2, C2, GK3P , IFIT2, IFITM1, KREMEN1 and PDE4D, in particular, calculating the value as defined in steps a) and b) according to the method described above;
c) determining in a sample of said individual the expression level of the genes BATF2, GBP5, IFITM1, IL27, KCNJ2-AS1, SERPING1, STAT1, TNFRSF21 and VAMP5;
d) calculating whether the therapy is completed or not based on the value, the score and the expression level of the genes BATF2, GBP5, IFITM1, IL27, KCNJ2-AS1, SERPING1, STAT1, TNFRSF21 and VAMP5;
e) determining whether the therapy-end is reached or the therapy has to be continued.

That is, the inventors have recognized that based on the expression levels of the identified genes, in particular, on the basis of the expressed RNA, it is possible to correlate with treatment responses, to predict individual patient outcome and recurrence of disease as well as to determine the therapy-end of a treatment of tuberculosis infection accordingly.

Thus, individualization of the therapy is possible and personalization of the therapy allows increasing compliance of therapy as well as the wellbeing of the patients. Moreover, individually recognizing the therapy-end decreases the risk of development of resistance against the therapeutic agent and decreases the environmental burden with the agents accordingly.

The methods according to the present invention are particularly useful for tuberculosis being MDR-TB.

In another aspect, the invention concerns the use of a test kit or the use of a kit of parts for diagnosing or predicting the clinical outcome of the tuberculosis infection, for treatment monitoring and determining treatment duration and/or determining the therapy-end for the tuberculosis treatment, respectively, whereby said kit comprises means for determining the expression level of said genes as well as instructions of how to use the test kit or the kit of parts in a method according to present invention.

The means for determining the expression level of said genes are typical means known to the skilled person, e. g. nucleic acid application either on RNA or DNA level.

Moreover, the present invention provides an array comprising means for detecting the expression level of the genes CD274, FAM20A, GYG1, HIST1H1B, LPCAT2, TRIM27, RPAP3, A_33_P3281041, BATF2, C2, GK3P, IFIT2, KREMEN1, PDE4D, GBP5, IFITM1, IL27, KCNJ2-AS1, SERPING1, STAT1, TNFRSF21 and VAMP5.

In another aspect, the present invention relates to a computer-implemented method of diagnosing or predicting the clinical outcome of the tuberculosis infection, for treatment monitoring and determining treatment duration, and/or for determining the therapy-end of tuberculosis infection, respectively, comprising the steps of:

a) obtaining data of measured expression level of the genes as defined in methods described above from a sample of an individual and, optionally, of controls;
b) computing the data of step a) by comparing the score and/or value and/or said expression level of the genes in said sample with the score and/or value and/or said expression level of the genes obtained from a database or obtained as a measured level of a cut off control;
c) diagnosing or predicting the clinical outcome of the tuberculosis infection, treatment monitoring and determining treatment duration, and/or determining the therapy-end for the tuberculosis treatment, respectively,
d) optionally further comprising the step of showing the data of diagnosing or predicting the clinical outcome of the tuberculosis infection, treatment monitoring and determining treatment duration, and/or determining the therapy-end of tuberculosis infection, respectively, on an output unit, in particular showing the results of the diagnosis or the therapy-end for the tuberculosis treatment.

Finally, the present invention provides a computer readable medium or computer program product having computer executable instructions for performing the steps of the methods according to the present invention.

Brief description of the drawings

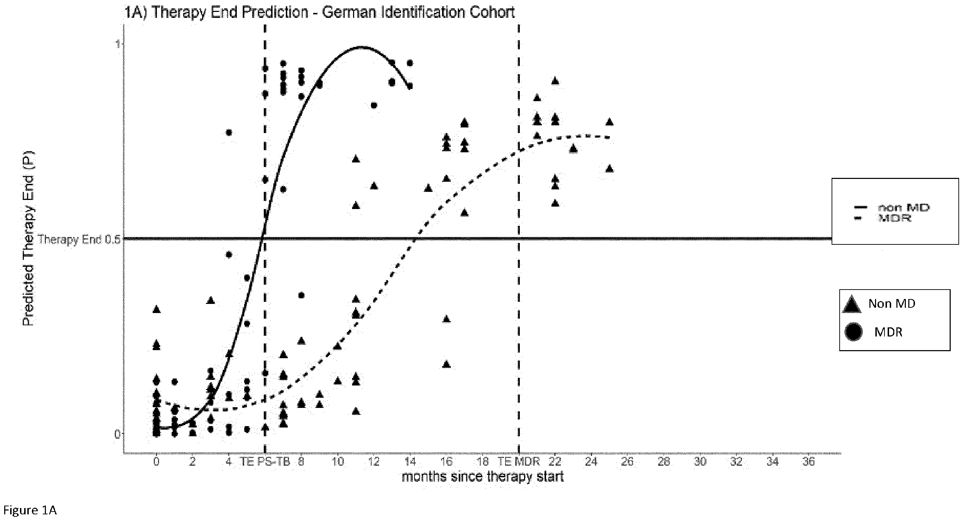

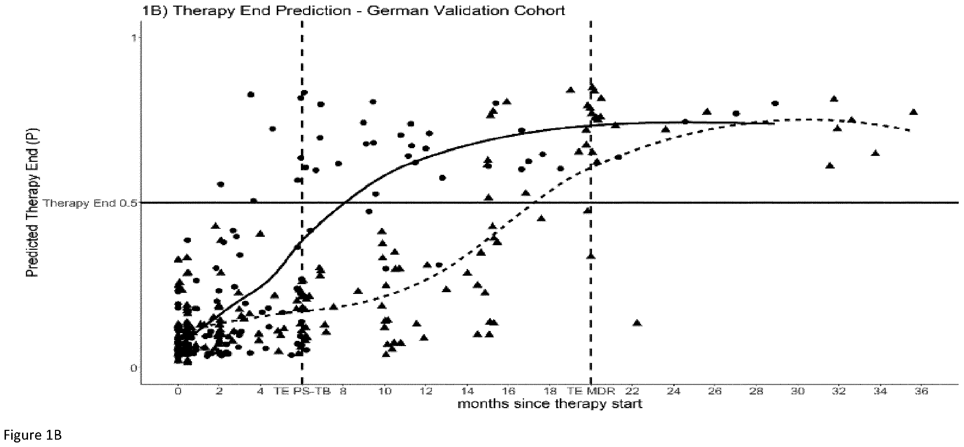

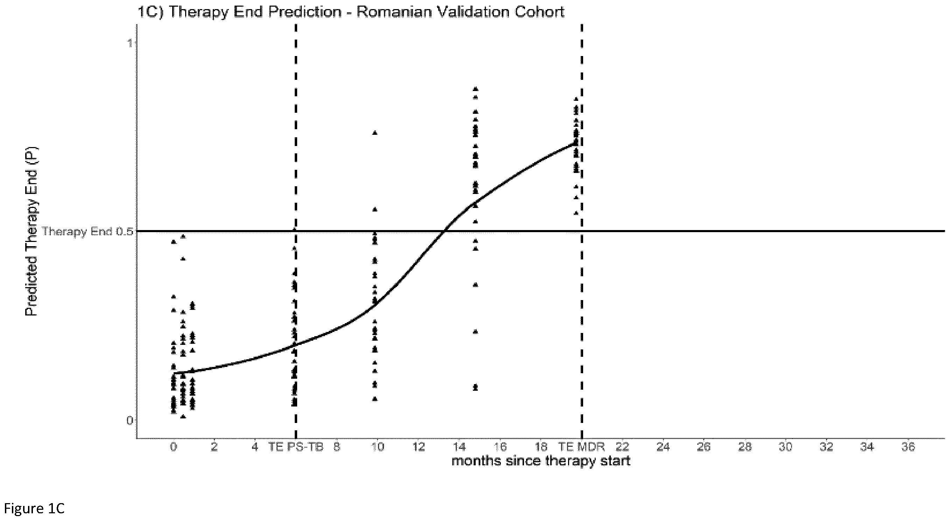

Figure 1: **1A:** Therapy end prediction in all patients of identification cohort. MDR = multidrug- and extensively drug-resistant tuberculosis. Non-MDR = susceptible and mono-resistant tuberculosis, TE PS-TB = planned end of therapy for patients with susceptible tuberculosis (6 months), TE MDR = planned end of therapy for patients with multidrug- and extensively drug-resistant tuberculosis (20 months). **1B:** Therapy end prediction in all patients of German validation cohort. **1C:** Therapy end prediction in all patients of Romanian validation cohort (only MDR patients).

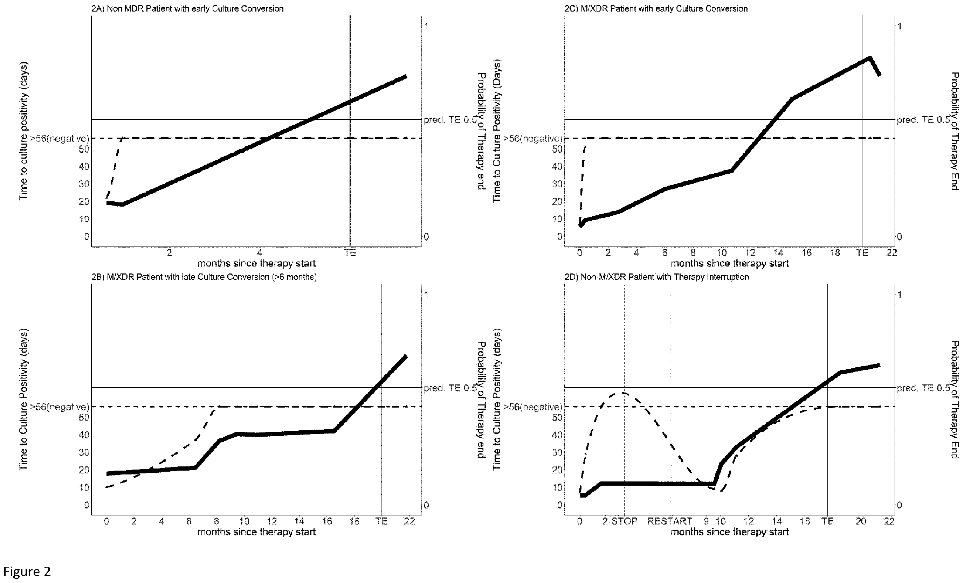

Figure 2: **2A:** Individual therapy prediction course (straight line) and time to culture positivity (dashed line) for non-MDR patient from German validation cohort with early culture conversion and an observed therapy duration of 6 months. TE = observed therapy end. pred.TE = threshold value for classification of predicted therapy end. **2B:** Individual therapy prediction course (straight line) and time to culture positivity (dashed line) for M/XDR patient from German validation cohort with observed therapy duration of 20 months. **2C:** Individual therapy prediction course (straight line) and time to culture positivity (dashed line) for M/XDR patient from German validation cohort with early culture conversion and an observed therapy duration of 20 months. **2D:** Individual therapy prediction course (straight line) and time to culture positivity (dashed line) for non-MDR patient from German validation cohort with therapy interruption of three months due to missing compliance and an observed overall therapy duration of 18 months.

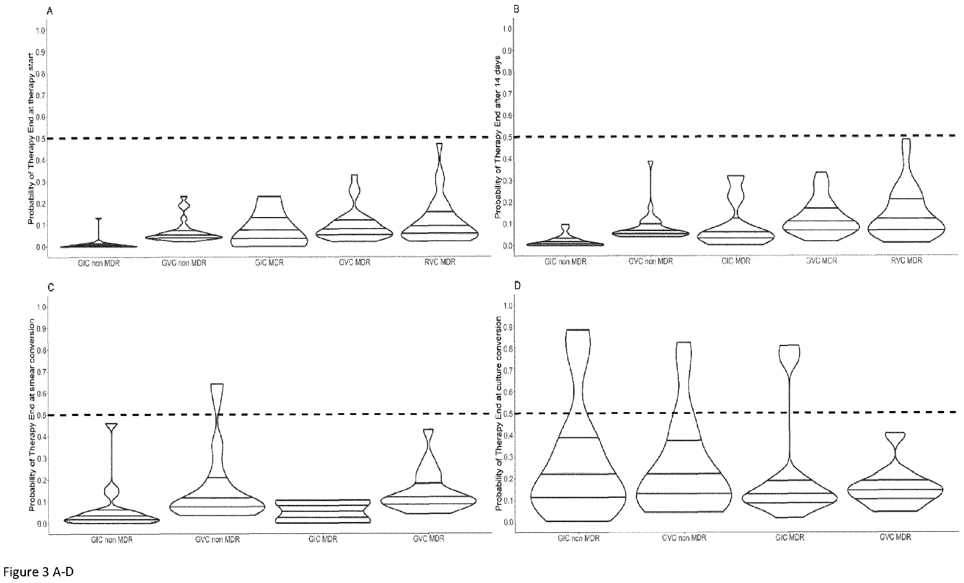

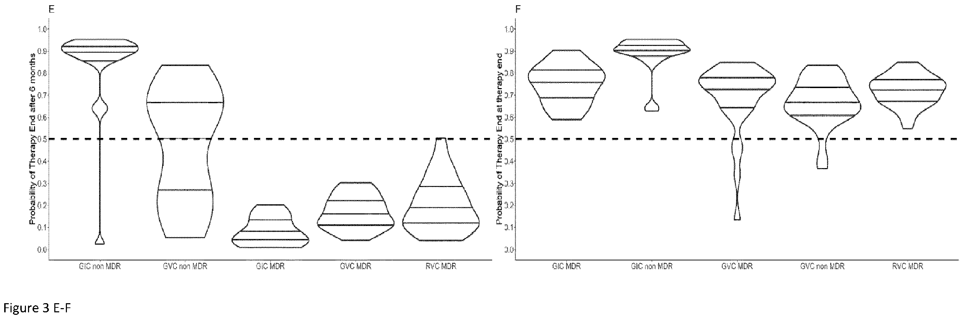

Figure 3: Comparison of the calculated therapy end probabilities between the cohorts at different times. **3A:** at therapy beginning, **3B:** after 14 days of therapy **3C:** at smear conversion, **3D:** at culture conversion **3E:** at first measurement after six months **3F:** after successful completed therapy.

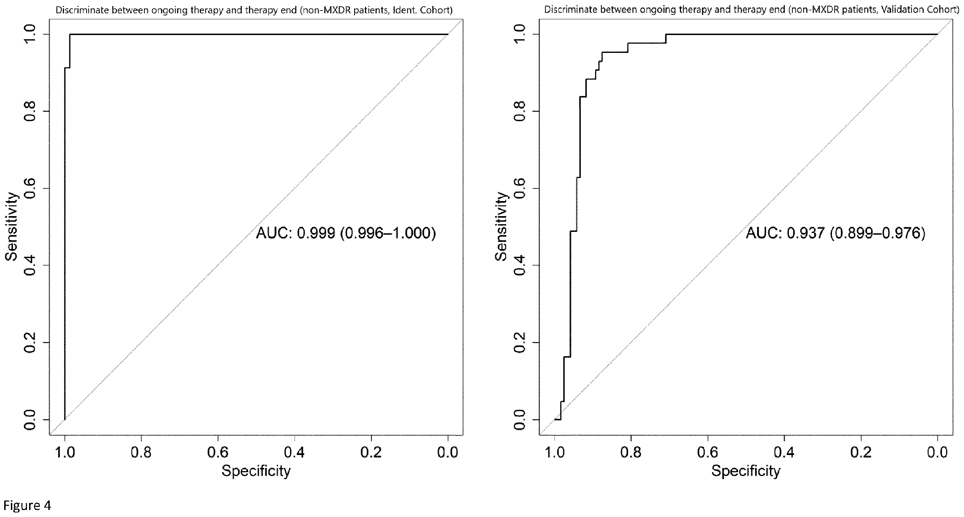

Figure 4: ROC curves, **4A:** Classification performance of transcriptomic rate for discriminating between ongoing therapy and therapy end in test-set of non-M/XDR patients from identification cohort, Area under the curve (AUC) = 0.999 (CI 0.996 - 1). **4B:** Classification performance of transcriptomic rate for discriminating between ongoing therapy and therapy end all non-MDR patients from German validation cohort, Area under the curve (AUC) = 0.912 (CI 0.868 - 0.956).

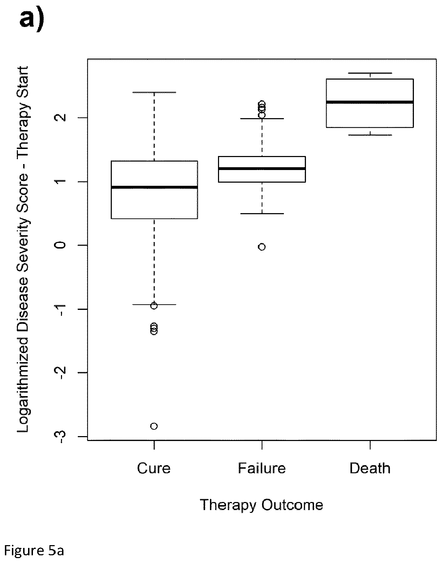

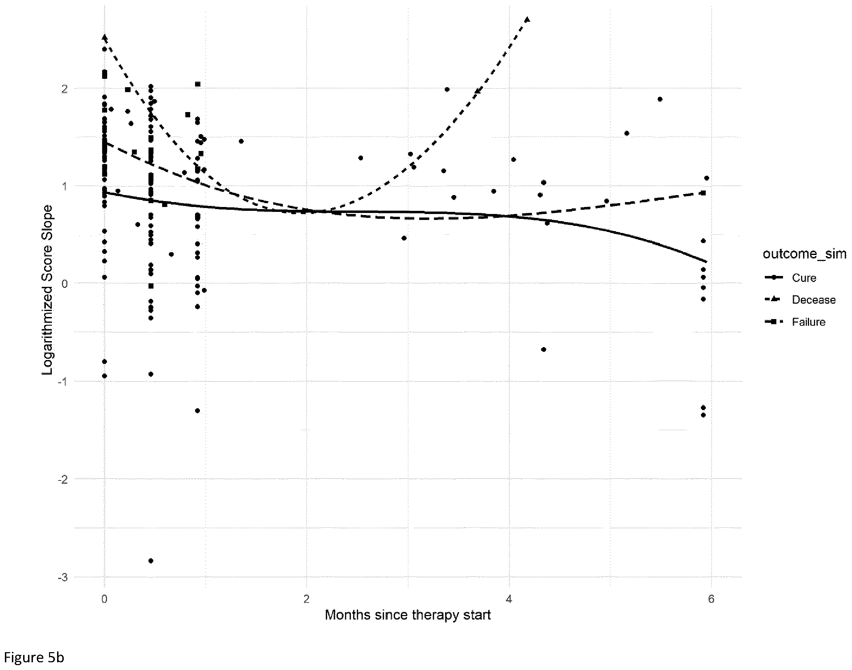

Figure 5: **5a:** Boxplot - Comparison of Disease Severity Score value in therapy naive patients with and without successful therapy outcome. **5b:** Logarithmic therapy response score values following course of therapy in merged datasets for outcome groups: Cure (straight, dots), Failure (dashed box), Death (slim dashed, triangles).

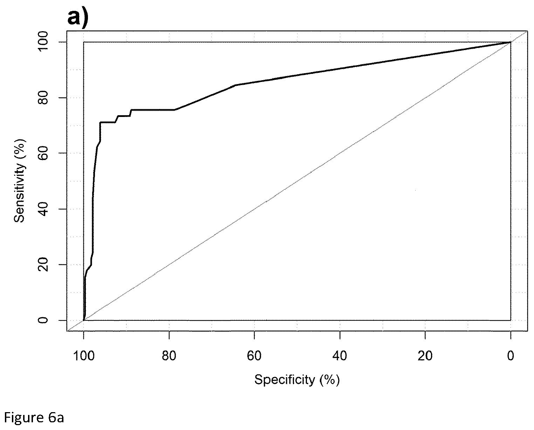

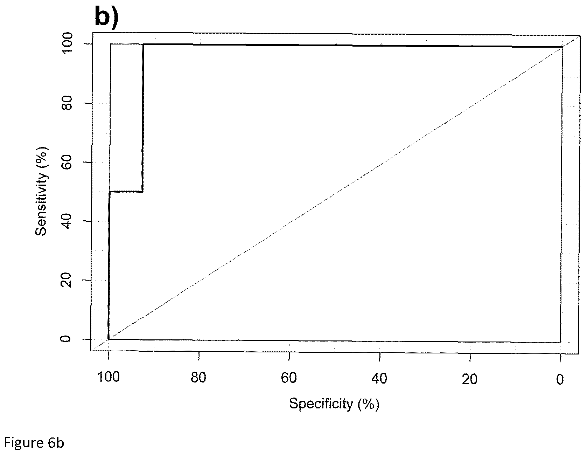

Figure 6: Receiver operator characteristics (ROC) curves showing the accuracies of the Disease Severity Score (DSS) for the discrimination between **6a:** cure and therapy failure during the whole course of therapy (AUC= 0.85, CI=0.78-0.92). **6b:** Surviving and death during the whole course of therapy (AUC= 0.96, CI=0.88-1).

Detailed description of the present invention

In a first aspect, the present inventions relates to a method for diagnosing or predicting the clinical outcome of tuberculosis infection in an individual afflicted with or suspected to be afflicted with a tuberculosis infection, comprising

a) determining the expression level of the genes: CD274, FAM20A, GYG1, HIST1H1B, LPCAT2 and TRIM27 in a sample obtained from said individual;
b) calculating a score based on the expression level of said six genes identified above;
c) diagnosing or predicting the clinical outcome of tuberculosis infection based on the score calculated in step b).

The inventors recognized that based on the expression level of the genes mentioned, namely CD274, FAM20A, GYG1, HIST1H1B, LPCAT2 and TRIM27, a score can be calculated which allows to diagnose or predict the clinical outcome of tuberculosis infection based thereon.

In this connection, the term "predicting the clinical outcome of tuberculosis infection" refers to the treatment failure or treatment cure as defined by the simplified outcome criteria, see e.g. the New England Journal of Medicine, 2016, 375, 11.

That is, based on this score, it is possible to determine treatment completion and differentiating the same with a treatment failure. In this connection, cure is defined as a negative culture status 6 months after treatment initiation, no positive culture thereafter, and no relapses within 1 year after treatment completion. Treatment failure is defined as a positive culture status 6 months after treatment initiation or thereafter or a relapse within 1 year after treatment completion. An undeclared outcome is defined as an outcome that was not assessed (owing to transferal out of the cohort, no culture status at 6 months while the patient was receiving care, or no posttreatment assessment). Death was defined as death during observation period. Loss to follow-up was defined as non-receipt of care 6 months after treatment initiation.

The method based on the expression level of the six genes mentioned above allows to predict final treatment outcome namely, cure or failure at different time points in the course of therapy.

In a particular embodiment, the expression level of the genes is based on determining the expression level of RNA with known methods. For example, a disease severity score (DSS) is determined. Said score weights the expression level of at least of the six genes. A specific embodiment is shown in the following formula:

$$Score = -62.19940 + CD274 * 0.70954 + FAM20A * 0.09335 + GYG1 * 1.68156$$
$$+ HIST1H1B * 3.71256 + LPCAT * 1.18746 + TRIM27 * 0.142288.$$

In another aspect, the present invention relates to a method for treatment monitoring and determining treatment duration of an individual with tuberculosis infection, comprising the steps of:

> a) determining the expression level of the genes: RPAP3, A_33_P3281041, BATF2, C2, GK3P, IFIT2, IFITM1, KREMEN1 and PDE4D in a sample obtained from said individual;
> b) calculating a value based on the expression level of said genes identified in step a);
> c) determining the therapeutic regimen and/or the treatment course in said individual based on the value obtained in step b).

The method is particularly useful for predicting the remaining days of therapy.

Namely, the value obtained according to the method, including the expression level of the genes RPAP3, A_33_P3281041, BATF2, C2, GK3P, IFIT2, IFITM1, KREMEN1 and PDE4D, allows to determine and predict the therapy-end and to monitor treatment and to determine treatment duration, accordingly.

It is noted that the terms "treatment monitoring" and "therapy monitoring" are used interchangeably herein.

Based on the expression level, the value is calculated. In a preferred embodiment, the expression levels of said genes are weighted. In particular, the weighting is conducted based on the following formula resulting in a value. Said value expresses the days till therapy end.

$$Days\ till\ therapy\ end = (-3170.49 + RPAP3 * 113.27 + A\_33\_P3281041 *$$
$$275.53 + BATF2 * 86.86 + C2 * -57.62 + GK3P * -102.19 + IFIT2 * -101.94 +$$
$$IFITM1 * 73.64 + KREMEN1 * 75.52 + PDE4D * 76.23 + MDR * 235.74) -$$
$$Days\ since\ therapy\ start$$

In this connection, the numerical value for the presence or absence of MDR-TB is 1 or 0, respectively, namely in case of MDR-TB the numerical value is 1, while in the absence of MDR-TB, the numerical value is 0.

Based on said calculation, the treatment of said individual can be monitored and treatment duration including the time remaining till therapy-end of said individual can be determined accordingly.

In a further aspect, the present invention relates to a method for determining the therapy-end of a treatment of tuberculosis infection in an individual afflicted with tuberculosis infection, comprising:

> a) determining in a sample obtained from said individual the expression level of the genes CD274, FAM20A, GYG1, HIST1H1B, LPCAT2 and TRIM27, preferably, calculating the score according to the method described above;
> b) determining in a sample of said individual the expression level of the genes RPAP3, A_33_P3281041, BATF2, C2, GK3P , IFIT2, IFITM1, KREMEN1 and PDE4D, in particular, calculating the value as defined in steps a) and b) of the method described above;
> c) determining in a sample of said individual the expression level of the genes BATF2, GBP5, IFITM1, IL27, KCNJ2-AS1, SERPING1, STAT1, TNFRSF21 and VAMP5;
> d) calculating whether the therapy is completed or not based on the value, the score and the expression level of the genes BATF2, GBP5, IFITM1, IL27, KCNJ2-AS1, SERPING1, STAT1, TNFRSF21 and VAMP5;
> e) determining whether the therapy-end is reached or the therapy has to be continued.

This method includes the determination of the score as defined in the above method for diagnosing the clinical outcome of tuberculosis infection. Further, the method takes into consideration the expression level of additional genes, namely, the expression level of the genes BATF2, GBP5, IFITM1, IL27, KCNJ2-AS1, SERPING1, STAT1, TNFRSF21 and VAMP5.

Based on this, it can be determined whether the end of therapy has been reached or the therapy must be continued.

Namely, the method for determining the end of therapy of the treatment of tuberculosis infection in an individual identifies positively whether the therapy can be concluded or has to be continued.

In an embodiment of the present invention, the methods according to the present invention as described above are conducted with a sample of said individual selected from sputum, blood, including whole blood, plasma and serum. In a preferred embodiment, the sample obtained from said individual is whole blood.

This is in contrast to the prior art methods. Namely, the prior art describes the determination of respective markers from sputum, the present method is particularly useful for determining the expression level of said genes in whole blood, in particular, determining the transcription rate accordingly.

That is, while the production of sputum and thus the informative value of a sputum-based biomarker decreases during TB therapy, the determination of the expression level of the referenced genes as biomarker from whole blood remains stable and it is hence possible to determine these biomarkers over the entire period of treatment.

This is particularly true for individuals afflicted with MDR-TB.

The method according to present invention allows determining individual therapy end time points with high accuracy. Thus, it is possible to individualize the therapy, to avoid unnecessary burden on the patient otherwise caused by the toxicity of the therapeutic (i.e. antimycobacterial) agents, and to reduce the risk of resurrection of infection as well as the development and propagation of resistant pathogens.

Moreover, the method as described herein, namely, the method for diagnosing or predicting the clinical outcome of tuberculosis infection allows to determine cure or failure of a therapy at different time points, thus, enabling the attending physician to identify whether the individual needs a further therapy, or whether said individual has been cured.

The method of the present invention represents the first biomarker-based method that allows the individual determination of the therapy period and the therapy-end not only for TB, but also for MDR-TB. That is, regardless of the status of resistance of the MTB pathogenic strain, the therapy-end as well as the clinical outcome can be predicted.

Without bound to theory, it is submitted that the method according to present invention and the model underlying this method are based primarily on the immune response of the host but not of the presence of active pathogens. Rather, the method allows to have personalized and individual monitoring of the therapy success as well as prediction of the end of therapy. The duration of the therapy may depend of course also on other factors, including bacterial load, the kind of resistance, availability of drugs as well as the general physical constitution of the patient. However, the method according to the present invention, as well as the model underlying the same allow predicting and determining the respective information, including the end of therapy, more independently of the above mentioned factors, namely, bacterial and clinical factors.

Moreover, in particular when using whole blood as sample obtained from the individual, it is possible to conduct the methods according to present invention as point of care methods for the management of the tuberculosis infection, in particular infection with MDR strains accordingly.

In an embodiment of the present invention the expression of the genes is determined based on the level of RNA present in the sample. Namely, in an embodiment the transcription level of said genes is determined based on RNA expression. For example, RNA expression in whole blood obtained from patients is determined.

In another embodiment, the methods of the present invention comprise an amplification step of nucleic acids, in particular RNA or cDNA obtained from said RNA after reverse transcription. That is, the amplification of the nucleic acids is conducted by known methods.

Moreover, in a further embodiment, the present invention relates to methods wherein in determining the course of treatment, the period until the end of therapy is predicted. That is, based on the value obtained by determining the expression level of the genes RPAP3, A_33_P3281041, BATF2, C2, GK3P, IFIT2, IFITM1, KREMEN1 and PDE4D, it is possible to predict the period till end of therapy. Particularly, it is possible to apply the following formula:

$$Days\ till\ therapy\ end = (-3170.49 + RPAP3 * 113.27 + A\_33\_P3281041 * 275.53 + BATF2 * 86.86 + C2 * -57.62 + GK3P * -102.19 + IFIT2 * -101.94 + IFITM1 * 73.64 + KREMEN1 * 75.52 + PDE4D * 76.23 + MDR * 235.74) -$$

$$Days\ since\ therapy\ start$$

In case of the presence of multidrug-resistant tuberculosis strains, the numerical value for MDR is 1, while in the absence of MDR tuberculosis strains, the numerical value of MDR is 0.

Further, the method according to present invention for determining the therapy-end for a treatment of tuberculosis infection in an individual afflicted with tuberculosis infection, comprises:

a) determining in a sample of said individual the expression level of the genes CD274, FAM20A, GYG1, HIST1H1B, LPCAT2 and TRIM27, preferably, calculating the score according to the method described above;

b) determining in a sample of said individual the expression level of the genes RPAP3, A_33_P3281041, BATF2, C2, GK3P , IFIT2, IFITM1, KREMEN1 and PDE4D, in particular, calculating the value as defined in steps a) and b) of the method described above;

c) determining in a sample of said individual the expression level of the genes BATF2, GBP5, IFITM1, IL27, KCNJ2-AS1, SERPING1, STAT1, TNFRSF21 and VAMP5;

d) calculating whether the therapy is completed or not, based on the value, the score and the expression level of the genes BATF2, GBP5, IFITM1, IL27, KCNJ2-AS1, SERPING1, STAT1, TNFRSF21 and VAMP5;

e) determining whether the end of therapy has been reached or if the therapy has to be continued;

may be subject to Random Forest (RF) classifications with 5,000-fold iterations, which results in average probability values for cure associated end of therapy. That is, in an embodiment, the calculation includes a random RF classification with a predetermined amount of iterations.

For example, the threshold value for the successful end of therapy applying RF classification is set at p < 0.5 (standard RF classification threshold). Based thereon, it is possible to define individual therapy end points with high accuracy for MDR-TB patients, as well as non-MDR-TB patients accordingly.

In a further embodiment of the present invention, the tuberculosis the individual is afflicted with or suspected to be afflicted with is a multidrug-resistant (MDR) tuberculosis.

In a further aspect, the present invention relates to the use of a test kit or the use of a kit of parts for diagnosing or predicting the clinical outcome of the tuberculosis infection, for treatment monitoring and determining treatment duration, and/or for determining the therapy-end for the anti-tuberculosis treatment, respectively. Said kit or kit of parts comprises means for determining the expression level of the genes identified in the method according to present invention as well as instructions of how to use said test kit or kit of parts for a method according to present invention.

The skilled person is well aware of suitable means for determining the expression level of a gene. Said means include components suitable for amplification of nucleic acids including RNA and DNA. For example, in an embodiment of the present invention the test kit or kit of parts includes an amplification kit for amplification of nucleic acids.

Moreover, the test kit or kit of parts may include components for prior purification or treatment of the sample obtained from said individual to be tested. Said compounds or components include means for preparing and providing RNA for further use. Said means include enzymes and suitable buffer system to isolate or purify RNA while other cell compartments are separated.

Further, the test kit or kit of parts according to the present invention includes means for detecting the amplification products or the expression level of said genes generally. Said means may include suitable probes, which may be labelled by suitable labels or marker molecules. Alternatively, the amplification products may be labelled based on using labelled modified nucleic acid molecules. The skilled person is well aware of suitable methods for performing amplification labelling accordingly, e. g. using expression microarrays, next generation sequencing etc.

Moreover, an array, such as a microarray or next generation sequencing, is provided, comprising means for detecting the expression of the genes CD274, FAM20A, GYG1, HIST1H1B, LPCAT2, TRIM27, RPAP3, A_33_P3281041, BATF2, C2, GK3P, IFIT2, KREMEN1, PDE4D, GBP5, IFITM1, IL27, KCNJ2-AS1, SERPING1, STAT1, TNFRSF21 and VAMP5.

Suitable means are available. In an embodiment, the arrays consists of means for detecting the expression of the genes CD274, FAM20A, GYG1, HIST1H1B, LPCAT2, TRIM27, RPAP3, A_33_P3281041, BATF2, C2, GK3P, IFIT2, KREMEN1, PDE4D, GBP5, IFITM1, IL27, KCNJ2-AS1, SERPING1, STAT1, TNFRSF21 and VAMP5.

Said array or next generation sequencing may be provided in form of a microarray or other suitable platforms for determining and detecting the expression level of said genes accordingly.

In an embodiment, the present invention relates to the use of the test kit or the kit of parts or the array as described herein in a method according to the present invention.

In another aspect, the present invention relates to the *in vitro* use of the genes as defined herein in diagnosing or predicting the clinical outcome of the tuberculosis infection for treatment monitoring and determining treatment duration, and/or determining the therapy-end of tuberculosis infection, respectively, by determining the expression level of said genes in a sample of an individual afflicted with or suspected to be afflicted with tuberculosis infection and comparing the expression level of the corresponding values and/or scores derived thereof with reference values including reference pattern and reference profiles.

Typically the reference value is obtained from non-infected individuals and/or from individuals with confirmed infection with MDR-TB or non-MDR/-TB.

Further, the present invention relates to a computer implemented method of diagnosing or predicting the clinical outcome of the tuberculosis infection, treatment monitoring and determining treatment duration, and/or determining the therapy-end for the tuberculosis treatment, respectively, comprising the steps of:

a) obtaining data of measured expression level of the genes as defined in the methods according to the present invention from a sample of an individual and, optionally, of controls;
b) computing the data of step a) by comparing the scores and/or values and/or said expression levels of the genes in said sample with the scores and/or values and/or said expression levels of the genes obtained from a database or obtained as a measured levels of predetermined cut-off controls;
c) diagnosing or predicting the clinical outcome of the tuberculosis infection, treatment monitoring and determining treatment duration, and/or determining the therapy-end of tuberculosis infection, respectively,
d) optionally further comprising the step of showing the data of diagnosing or predicting the clinical outcome of the tuberculosis infection, of treatment monitoring and determining treatment duration, and/or of determining the therapy-end for the tuberculosis treatment, respectively, on an output unit, in particular showing the results of the diagnosis of tuberculosis infection or the therapy-end for the tuberculosis treatment.

In step b), the data may be computed by comparing the scores of the individual to be analyzed with scores, values or expression levels of said genes obtained from a database or obtained as a measure to levels of cut-off controls.

For example, the computer-implemented method, designated for obtaining the data of measured expression level, comprises the classification with the Random Forest classifications with x-fold iterations, for example 1,000-fold iterations, like 3,000-fold iterations, e. g. 5,000-fold iterations.

Further, other calculation measures include: General linear regression model (GLM), naive bayes, bayesian belief network, multinomial naive bayes, gaussian naive bayes, bayesian network, averaged one-dependence estimators, logistic regression model, elastic net model, classification and regression trees (CART), iterative di-chotomiser 3, C4.5, C5, M5, chi squared automatic interaction detection, conditional decision tree, decision stump, support vector machines (svm), hopfield network, perceptron, multilayer perceptrons, radial basis function, stochastic gradient descent, back propagation, k-nearest networks (kNN), k-means, k-medians, hierachical clustering, expectation maximisation, lasso regression, ridge regression, least angle regression, eclant algorithm, a priori algorithm, gradient boosting algorithms, learning vector quantization, self-organization map, locally weighted learning, convolutional neural networks, stacked auto-encoders, recurrent neural network, long short term memory network, deep belief network, deep bolzmann machine, PCA, Principal component regression, projection pursiut, PLSR, sammon mapping, multidimensional scaling, linear discriminant analysis, mixture discriminant analysis, flexible disc. analys., bagging, boosting, adaboost, blending, gradient boosted regression tree, stacking, genetic optimization algorithm.

The method runs on a suitable data processing unit. Said data processing unit may contain an output unit that allows the results of the diagnosis of tuberculosis infection or the therapy-end for the tuberculosis treatment to be displayed, as well as to give further information to the individual. The data processing unit may be connected to a database storage unit or a database retrieval unit, which allows the comparison of the data of the measured expression level of the genes according to present invention, e. g. given as a score or value as described herein with the score and/or value and/or expression level of said genes obtained from other individuals and/or cohorts of individuals.

Finally, the present invention relates to a computer readable medium or computer program product having computer executable instructions for performing the steps of the methods according to present invention, including the computer-implemented method as described herein.

The present invention will be described further by way of examples, but without limiting the same.

**Examples**

**MATERIALS AND METHODS**

Study Cohort

Between March 2013 and March 2016, patients with pulmonary TB (non-M/XDR-TB and M/XDR-TB), identified by detection of *M. tuberculosis* DNA from sputum by the GeneXpert MTB/RIF test (Cepheid, Sunnyvale, USA), were prospectively enrolled in the identification cohort at the Medical Clinic, Research Center Borstel; Karl-Hansen-Klinik, Bad Lippspringe; Sankt Katharinen-Krankenhaus, Frankfurt; Thoraxklinik-Heidelberg, Heidelberg; Asklepios Fachkliniken München-Gauting, Munich in Germany as described previously (Heyckendorf, J., et al., Eur Respir J 51(2018), Heyckendorf, J., et al., Int J Tuberc Lung Dis 22, 399-406 (2018)). Individuals were excluded, if they were younger than 18 years, under legal supervision, or if they were (co-)infected with HIV.

Between March 2015 and April 2018, patients with non-M/XDR-TB and M/XDR-TB were prospectively enrolled into the validation cohort at the same centers. In addition, patients with M/XDR-TB were prospectively enrolled for a second validation cohort at the Marius Nasta Institute (MNI) in Bucharest, Romania, between May 2015 and March 2017. Healthy controls (HC) with no history of previous TB and without any known current illnesses at the time point of sampling were enrolled between June 2015 and December 2015 at the Medical Clinic of the Research Center Borstel (Germany).

Study visits included clinical assessment and blood sampling (baseline before treatment initiation; at 14 days of therapy, time of smear conversion and culture conversion; at 6 months and/or therapy end in non-M/XDR-TB; and at 10, 15, 20 months of therapy in M/XDR-TB patients). An additional study visit after 4 weeks and no further sampling after month 10 of therapy were performed in patients from the second M/XDR-TB validation cohort in Romania. Culture and smear evaluations from sputum samples in Germany were performed at the National Reference center for Mycobacteria. Samples at the MNI were analyzed at the Romanian National Reference Center for Mycobacteria. The anti-TB therapy regimens were based on comprehensive drug-susceptibility testing and on the basis of current therapy recommendations. Therapy outcomes were assessed following the TBNET simplified and the WHO therapy outcome definitions (Gunther, G., et al., N Engl J Med 375, 1103-1105 (2016), World Health Organization. Definitions and reporting framework for tuberculosis - 2013 revision (updated December 2014), (WHO Library Cataloguing-in-Publication Data, Geneva, Switzerland, 2014)).

RNA isolation and Quality Control

Whole blood RNA from Paxgene tubes (Qiagen®, Venlo, the Netherlands) were handled according to the manufacturer's instructions and stored at -80°C until RNA isolation using the Paxgene blood RNA isolation kit (Qiagen®, Venlo, the Netherlands). Aliquots of isolated RNA were used for quality control to analyze the RNA integrity with the RNA Nano 6000 Kit on an Agilent Bioanalyzer (Agilent®, Böblingen, Germany) according to the manufacturer's instructions. In case of an insufficient RIN number or signs of degradation, samples were excluded from further analysis.

Labeling, hybridization and scanning of microarrays

Total RNA was used for reverse transcription and subsequent Cy3-labelling with the Low-Input Quick Amp Labeling Kit (Agilent®, Böblingen, Germany) according to the One-Color Microarray-Based Gene Expression Analysis Protocol version 6.9.1 (Agilent®, Böblingen, Germany) with RNA Spike-In controls. 1,650 ng of Cy3-labelled cRNA was hybridized to human 4x44K V2 gene expression microarrays according to manufacturer's instructions. Arrays were scanned on a SureScan microarray scanner (Agilent®, Böblingen, Germany) at a resolution of 5 μm.

Data extraction and normalization

Raw expression data from scanned microarray slides was extracted from tiff files using the Feature Extraction Software version 11 (Agilent®, Böblingen, Germany). Raw data files were imported into Agilent GeneSpring software version 13 (Agilent®, Böblingen, Germany). Percentile Shift was used as normalization method with the 75th percentile as target and baseline transformation was applied to the median of all samples. Prior to data analysis, quality control was performed and compromised probes removed from further analysis. "Normalized expression" refers to Log2 transformed expression values.

Microarray data analysis

The data analysis and model development followed a multi-step approach. First, genes that characterized the severity of disease were identified by a moderated T-test with a fold-change cut-off of ≥2 and a Benjamini-Hochberg multiple testing correction of p ≤0.01 in healthy controls compared to therapy-naive patients from the training set. Hierarchical clustering was run on samples and entities with a Pearson Centered similarity rule and Ward's Linkage rule. Correlation was calculated according to Pearson's correlation coefficient. Analysis of gene lists for enriched pathways was done via the web-interface of g:Profiler (https://biit.cs.ut.ee/gprofiler/). Here, Kegg and Reactome pathways were chosen from the curated gene sets with a p-value cut-off for multiple corrections below 0.05. A first sub-model for calculating the DSS was created from these genes. A second sub-model was developed for calculation of the remaining days of therapy. For this, transcripts that correlate with remaining therapy duration were identified and corrected for other influencing genes. From this, a model was created to calculate the remaining days until the end of therapy for each patient at any time. As next step, additional genes were also identified, which were differently expressed during ongoing therapy and after therapy end. This was done with L1 regularization. These genes and the two variables previously created, which describe the severity of the disease and the remaining therapy time, were the starting point for the final model. The further variable reduction was mainly based on the Akaike Information Criterion (AIC) and on the comparison of the reduced models using ANOVA. The final reduced model provides the end-of-treatment probabilities for each patient for each measurement.

**RESULTS**

Seventy-nine patients (20 M/XDR TB; 59 non M/XDR TB) from an identification cohort in Germany, 60 patients (28 M/XDR TB; 32 non M/XDR TB) from the validation cohort in Germany, and 52 patients (all M/XDR TB) from a validation cohort in Romania with confirmed pulmonary TB were enrolled in this study (Table 1).

Table 1: Characteristics of TB patients, observed and predicted therapy durations in patients from the German identification cohort (GIC), the German validation cohort (GVC), and the Romanian validation cohort (RVC). Sd = standard deviation, TTC+ = time to culture positivity, M/XDR-TB = multidrug-resistant and extensively drug-resistant tuberculosis.

| | German identification cohort (n=79) | German validation cohort (n=60) | Romanian validation cohort (n=52) | p-value |
| --- | --- | --- | --- | --- |
| Baseline Age (mean, sd) | 41.8 (+/-15.9) | 36.7 (+/-13.9) | 37.0 (+/-11.2) | 0.055 |
| Sex male | 48 (60%) | 41 (68.3%) | 33 (55.9%) | 0.652 |
| M/XDR-TB | 20 (43.5%) | 28 (53.3%) | 52 (100%) | <0.001 |
| Baseline TTC+ in days (mean, sd) | 22.5 (+/-16.1) | 27.9 (+/-21.8) | 28.4 (+/-15.1) | 0.101 |
| Time to culture conversion in days (mean, sd) | 67.1 (+/- 43.7) | 53.8 (+/- 36.5) | 57.6 (+/-40.6) | 0.141 |
| Observed therapy duration of non-M/XDR patients in days (mean, sd) | 258.2 (+/-92.7) | 275.2 (+/-85.4) | - | 0.417 |
| Observed therapy duration of M/XDR patients in days (mean, sd) | 623.2 (+/-33.9) | 648.8 (+/-97.3) | 591.7 (+/-64.2) | 0.008 |
| Predicted therapy duration of non-M/XDR patients in days (mean, sd) | 230.1 (+/-91.0) | 225.4 (+/-66.1) | - | 0.8903 |
| Predicted therapy duration of M/XDR patients in days (mean, sd) | 455.5 (+/-113.0) | 471.7 (+/-75.7) | 475.9 (+/-75.9) | 0.795 |

Baseline time to sputum culture positivity was not significantly lower in the identification cohort when compared to the validation cohorts (22.5 vs. 27.9 or 28.4 days, p=0.101). The mean duration of therapy in non-M/XDR patients from the identification cohort was 258.2 days (Standard deviation; SD +/- 92.7 days) and 275.2 days (SD +/-85.4 days, p=0.417) in the validation cohort in Germany. Patients with M/XDR-TB from the identification cohort were treated for a mean duration of 623.2 days (SD +/-33.9 days) while patients with M/XDR-TB from the validation cohort in Germany and Romania received anti-TB treatment for a mean of 648.8 days (SD +/- 97.3 days) and 591.7 days (SD +/- 64.2 days p=0.008), respectively.

Transcriptomic analysis

Disease Severity Score (DSS)

A model to predict final treatment outcome (cure vs. failure) involved the expression levels of six transcripts, i.e. CD274, FAM20A, GYG1, HIST1H1B, LPCAT2 and TRIM27. This model showed an excellent to outstanding performance in differentiating between patients with cure and those experiencing therapy failure at different time points in the course of therapy with an AUC of 0.85-1.0. Outcome data were also present for the second validation cohort were this model was able to differentiate therapy naive patients later developing either cure or failure with an AUC of 0.85 (0.55 - 1). As a result, an RNA based disease severity score (DSS, Figure 6) using the following formula was derived:

$$Score = -62.19940 + CD274*0.70954 + FAM20A*0.09335 + GYG1*1.68156 + HIST1H1B*3.71256 + LPCAT*1.18746 + TRIM27*0.14288.$$

Kruskal-Wallis Test showed highly significant score differences between outcome groups at baseline (p<0.001, Figure 5). Patients with cure had a mean score value of 2.90 (IQR: 1.41 - 4.06) before therapy initiation while patients with therapy failure exhibited a mean score value of 4.06 (IQR 2.90 - 10.67) and deceased patients a mean score value of 10.30 (IQR 7.23 - 13.69). The overall performance of the transcriptional rate and the DSS is shown in Figure 6. The DSS was later applied as a single numerical variable for the final therapy duration model.

Days till therapy end prediction

As the second step, an additional model predicting the days until end of successful therapy in non-M/XDR-TB patients and M/XDR-TB patients from the identification cohort was created. The pre-selection of genes for this model was based on the correlation between the gene's expression level and the days since therapy induction. Here, the expression level of additional nine genes, i.e. RPAP3, A_33_P3281041, BATF2, C2, GK, IFIT2, IFITM1, KREMEN1, and PDE4D, were suitable to describe 53% variance of the remaining therapy duration. The prediction of the remaining days of therapy is represented by the following formula: Days till therapy end = (-3170.49 + RPAP3*113.27 + A_33_P3281041 *275.53 + BATF2*86.86 + C2*-57.62 + GK3P*-102.19 + IFIT2*-101.94 + IFITM1*73.64 + KREMEN1*75.52 + PDE4D*76.23 +MDR*235.74)-Days since therapy start.
For M/XDR the MDR is 1 and for non-M/XDR the MDR is 0.

Prediction of therapy end

A third step was conducted to derive probabilities for successful therapy-end at any given time point of sampling. Therefore, lasso regression was performed throughout the gene-expression data set (cross validated train set of non-M/XDR-TB of the identification cohort) to identify genes corresponding with the actual end of therapy. This enabled the identification of genes of interest to characterize successful therapy end.
After extensive variable reduction procedures, the final method to define therapy duration consisted of the following additional genes: BATF2, GBP5, IFITM1, IL27, KCNJ2-AS1, SERPING1, STAT1, TNFRSF21, VAMP5, plus the DSS, and the predicted remaining days of therapy. These variables were used for random forest (RF) classifications with 5,000-fold iterations, which resulted in average probability values for cure associated end of therapy. The threshold value for the successful end of therapy was set at P>0.5 (standard RF classification threshold).
The method was able to define individual therapy end time points with an AUC of 0.999 (95% CI: 0.996-1.0, Figure 4a) in the test set of non-M/XDR patients from the identification cohort. For the non-M/XDR-TB patients of the first validation cohort from Germany, the AUC was 0.937 (95% CI: 0.899-0.976 (see Figure 4b)). This model was then applied to data from the three independent M/XDR-TB cohorts to calculate individual probabilities for therapy end. Figure 1a-c shows the probabilities for the different cohorts as a function of the resistance status and time under therapy. In the identification cohort, the first non-M/XDR-TB-patient is predicted to experience therapy end after 176 days of treatment, while the first M/XDR-TB-patient was indicated to require 195 days of treatment, for example. In the German validation cohort, the first non-M/XDR-TB-patient reaches the therapy end threshold after 107 days and after 182 days in an M/XDR-TB-patient.
Bacteriological time points such as the times of sputum culture and smear microscopy conversion with corresponding probabilities for successful therapy end were also compared between patients with non-M/XDR-TB and M/XDR-TB. No significant differences between non-M/XDR- and M/XDR-TB patients for these probabilities were observed although they occurred at later stages of therapy in M/XDR patients from the identification cohort (smear conversion: non-M/XDR-TB 63.2 days vs. M/XDR-TB 94.4 days, p=0.255; culture conversion: non-M/XDR-TB 134.9 days vs. M/XDR-TB 139.1 days, p=0.806.) and German validation cohort (smear conversion: non-M/XDR-TB 71.0 days vs. M/XDR-TB 77.3 days, p=0.855; culture conversion: non-M/XDR-TB 95.9 days vs. M/XDR-TB 96.0 days, p=0.993).
It is also noticeable that the predicted end of therapy was similar to the clinically conducted therapy ends in non-M/XDR-TB patients even if therapy had to be prolonged due to clinical therapy difficulties (i.e. late culture conversion or extensive disease).
In detail, 17/19 (89.5%) of non-M/XDR-TB patients from the GIC reached the marker threshold for cure following approximately 6 months (period 179-221 days) of therapy in comparison to 0/16 (0%) of M/XDR-TB patients. 24/32 (75%) of non-M/XDR-TB from the GVC cohort reached the marker's threshold for cure at their first measurement after 6 month (period 175-221 days) of anti-TB treatment while 0/28 (0%) patients with M/XDR-TB reached the threshold at this time point of therapy (period 175 - 318 days). The non-M/XDR patients not reaching the threshold after approximately 6 months showed a non-significant increase of the time to culture conversion (111.5 d vs 60.1 days, p= 0.292). In the RVC, one M/XDR patient (1/52 (1.9%)) was classified as cured by the model after 6 months of treatment (180 days), the proportion after approximately fifteen months of therapy was (3/20 (30%)) in the GIC, (6/28 (21.4%)) in the GVC, and (46/52 (88.5%)) in the RVC, and all M/XDR patients in the GIC, (27/28 (96.4%)) in the GVC, and all in the RVC following 20 months of therapy. Altogether, only one patient was classified as not having finished therapy after 20 months of therapy. Patients not reaching the probability threshold following the recommended therapy durations were characterized by extensive disease and cavitary lesions with high bacterial loads resulting in late culture conversions (see Table 2).

Table 2:

|  | Non M/XDR TB Patients - Calculated Therapy End within 6 months (n=45) | Non M/XDR TB Patients - Calculated Therapy End after 6 months (n=9) | p-value |
|---|---|---|---|
| Age | 37.3yr | 35.3yr | 0.771 |
| Male | 30 (66.6%) | 7 (77.8%) | 0.568 |
| BMI | 20.8 | 18.2 | 0.058 |
| Cavitaries:<br>   Cavitary<br>   Miliary | 24 (53.3%)<br>0 (0%) | 4 (44.4%)<br>1 (11.1%)) | 0.08 |
| Bilateral TB | 20 (44.4%) | 4 (44.4%) | 0.601 |
| Previous TB | 12(26.7%) | 6 (66.7%) | 0.023 |
| Time to culture Conversion | 60.1d | 111.5d | 0.292 |
| Initial Time to positivity | 21.3d | 16.5d | 0.192 |
| Active Smoking | 19 (43.2%) | 3 (33.3%) | 0.661 |

There were no relapse cases in all three cohorts.

The correlation coefficient between predicted and actual therapy duration in non-M/XDR patients from the identification and validation cohort was r=0.59. The predicted therapy duration based on the classification of the duration model did not differ significantly for non-M/XDR-TB patients of the identification cohort (230.1 days vs 258.2 days, p= 0.378) or the German validation cohort (225.4 days vs. 275.2 days, p=0.161). However, predicted durations were significantly lower in M/XDR-patients of the identification cohort (predicted 455.5 days vs. observed 623.2 days, p<0.001), the German validation cohort (predicted 471.7 days vs. observed 648.8 days, p<0.001) but not in Romanian validation cohort (predicted 475.9 days vs. observed 591.7 days, p=0.198). In the identification cohort, this would have resulted in a therapy duration that would have been 167.7 days less on mean. In the German validation cohort, therapy could have been 177.1 days shorter on average, whereas in the Romanian validation cohort, therapy could have been 115.8 days shorter on average.

The model was also suitable as treatment monitoring tool for individual patients (Figure 2a-d). The examination of the individual probability trajectories also showed a clear correlation between the time to culture positivity and the probabilities for the end of therapy. For example, the model indicated a therapy duration of approximately six months in a non-M/XDR-TB with early culture conversion from the German validation cohort as recommended by current guidelines (179 days predicted vs. 182 days conducted, Fig 2A). Correspondingly, an early culture converted M/XDR TB patient from the German validation cohort would have experienced a shorter therapy duration based on the duration model when compared to the standard WHO therapy duration that was clinically pursued (411 days predicted vs. 606 days conducted, Fig 2B). However, even an M/XDR-TB patient with late culture conversion (182 days) from the German validation cohort would have had a therapy duration of less than 20 months when defined by the duration model (502 days predicted vs. 607 days conducted, Fig 2C). The model also clearly corresponds to treatment interruptions, which was illustrated by a patient who experienced a three-month treatment discontinuation (Fig 2D). Here, treatment end probabilities remained at low levels and increased following therapy re-induction despite of negative cultures, that were observed intermittently. The predicted therapy duration in this specific case almost corresponded to the actually conducted therapy duration of 20 months (511 days predicted vs. 536 days conducted).

**DISCUSSION**

A model consisting of a 22-gene transcriptional signature was able to identify optimal therapy durations in three independent cohorts of non-M/XDR- and M/XDR-TB patients. The model was trained in German non-M/XDR-TB patients and re-assured in an independent non-M/XDR-TB German validation cohort also corresponding to actually conducted therapy durations with high accuracy. It yields probabilities for successful individual therapy ends at any given measurement, which were classified in multitude of machine learning iterations and correlates with culture based markers such as the time to culture positivity. The signature also has the potential to serve as therapy monitoring tool since probabilities for therapy end not reaching the critical threshold or changes of probabilities under therapy may indicate the need for ongoing therapy.

Transcriptional rates being predictive for the future development of active disease, correlating with active disease, or reflecting early responses to anti-TB therapy in active TB patients were described previously (Suliman, S., et al., Am J Respir Crit Care Med (2018), Zak, D.E., et al., Lancet 387, 2312-2322 (2016), Bloom, C.I., et al., PLoS ONE 7, e46191-e46191 (2012)). Although clinical therapy outcomes including relapse after therapy could also be predicted by certain RNA signatures studies, e.g. Bloom, C.I., et al., PLoS ONE 7, e46191-e46191 (2012), marker combinations to individualize therapy durations were missing. The present studies' results represent the first approach to biomarker-guided determination of individualized therapy durations in patients with M/XDR-TB. In contrast to previous studies (Darboe, F., et al.; Front Microbiol 10, 1441 (2019), Thompson, E.G., et al., Tuberculosis (Edinb) 107, 48-58 (2017)), the therapy duration model was able to predict successful individual therapy durations using established clinical endpoints (i.e. cure or failure) following the TBNET outcome definitions, which include a follow-up period of one year after therapy end to exclude for relapse.

Previous findings suggest TB specific changes to be mostly part of IFN-$\gamma$-signaling and related pathways, which can also be reproduced by this study. However, the transcriptional response to active *M. tuberculosis* infection in this study's TB patients appears to be independent of the pathogen's resistance status, which is essential, since the therapy duration model of this study was trained in non-M/XDR-TB patients to identify individual therapy durations in M/XDR-TB patients.

Therapy responses in patients with M/XDR-TB are usually evaluated by serial culture samples and the culture conversion status. However, sputum samples are often not accessible during later stages of therapy in most cases, which excludes culture as valid marker to individualize therapy durations. However, the therapy duration model showed plausible correlations with the TTP in individual assessments. Probabilities for therapy end at biographic events such as the time point of sputum smear or culture conversion were comparable in patients with non-M/XDR-TB and M/XDR-TB although they were reached at later stages of therapy in M/XDR-TB disease. These findings suggest that the hereby presented model reflects the host's inflammatory response to viable pathogens. The therapy duration model may be ideal to accompany and substitute for sputum culture evaluations at later stages of therapy as ideal therapy monitoring tool. A proper point-of-care assay system should be developed to conduct biomarker-guided therapies in M/XDR-TB patients in the future.

The shorter MDR-TB regimen is the attempt to treat patients with standardized therapy durations of 9-11 months. This regimen has been conducted in high numbers of patients with MDR-TB around the world leading to high rates of therapy success. However, *M. tuberculosis* isolates from European MDR-TB patients frequently carry 2nd-line drug-resistances also against the shorter course core drugs, which excludes the regimen's application in most European patients. Tailored therapies on the basis of comprehensive drug-resistance testing may be the more promising way to conduct effective drug therapies within these patients. The therapy duration model has added value to the individualized therapy approach since the drug regimens' effect can be monitored and individual durations may be precisely identified. Individualized durations largely depend on bacterial loads, the host constitution, the pathogens resistance pattern, and the availability of drugs. However, the duration model from this study works independently of any clinical (i.e. immune status) or pathogen characteristics and is based on RNA-expression levels only. The RNA-rate based individualized therapy durations may lead to less relapse cases, avoid adverse events in the case of shorter treatment durations, and reduce overall drug costs.

In conclusion, a host RNA-based model was able to identify optimal therapy durations in patients with M/XDR-TB. The RNA rate can be translated to a point-of-care test and therefore has the potential to change the future management of MDR-TB. The model has been validated sufficiently in this study to now conduct a prospective trial comparing standard of care with biomarker-guided durations.

**Claims**

1. A method for diagnosing or predicting the clinical outcome of tuberculosis infection in an individual afflicted with or suspected to be afflicted with a tuberculosis infection, comprising

    a) determining the expression level of the genes: CD274, FAM20A, GYG1, HIST1H1B, LPCAT2 and TRIM27 in a sample obtained from said individual;
    b) calculating a score based on the expression level of said six genes identified above;
    c) diagnosing or predicting the clinical outcome of tuberculosis infection based on the score calculated in step b).

2. A method for treatment monitoring and determining treatment duration of an individual afflicted with tuberculosis infection, comprising the steps of:

    a) determining the expression level of the genes: RPAP3, A_33_P3281041, BATF2, C2, GK3P, IFIT2, IFITM1, KREMEN1 and PDE4D in a sample from said individual;
    b) calculating a value based on the expression level of said genes identified in step a);
    c) treatment monitoring and determining treatment duration in said individual.

3. A method for determining the therapy-end of a treatment of tuberculosis infection in an individual afflicted with tuberculosis infection, comprising:

    a) determining in a sample of said individual the expression level of the genes CD274, FAM20A, GYG1, HIST1H1B, LPCAT2 and TRIM27, preferably, calculating the score according to claim 1 ;
    b) determining in a sample of said individual the expression level of the genes RPAP3, A_33_P3281041, BATF2, C2, GK3P , IFIT2, IFITM1, KREMEN1 and PDE4D, in particular, calculating the value as defined in steps a) and b) of claim 2;
    c) determining in a sample of said individual the expression level of the genes BATF2, GBP5, IFITM1, IL27, KCNJ2-AS1, SERPING1, STAT1, TNFRSF21 and VAMP5;
    d) calculating whether the therapy is completed or not based on the value, the score and the expression level of the genes BATF2, GBP5, IFITM1, IL27, KCNJ2-AS1, SERPING1, STAT1, TNFRSF21 and VAMP5;
    e) determining whether the therapy-end is reached or the therapy has to be continued.

4. The method according to any one of the preceding claims wherein the sample of said individual is selected from sputum, blood including whole blood, plasma and serum, preferably, the sample obtained from said individual is whole blood.

5. The method according to any one of the preceding claims wherein the expression of genes is determined based on RNA present in said sample.

6. The method according to any one of the preceding claims comprising an amplification step of nucleic acids, in particular RNA or cDNA obtained from said RNA after reverse transcription.

7. The method according to any one of the preceding claims wherein the tuberculosis is a multidrug resistant (MDR) tuberculosis.

8. The method according to any one of claims 2 to 7 wherein the value calculated according to claim 2 includes a factor of the presence or absence of MDR.

9. The use of a test kit or kit of parts for diagnosing or predicting the clinical outcome of the tuberculosis infection, for treatment monitoring and determining treatment duration, and/or determining the therapy-end for the tuberculosis treatment, respectively, said kit comprising means for determining the expression level of genes as identified in any one of claims 1 to 3 and instructions of how to use said test kit for a method according to any one of claims 1 to 8.

10. The use of a test kit or kit of parts according to claim 9 wherein said test kit or kit of parts includes an amplification kit for amplification of nucleic acids.

11. An array comprising means for detecting the expression of the genes CD274, FAM20A, GYG1, HIST1H1B, LPCAT2, TRIM27, RPAP3, A_33_P3281041, BATF2, C2, GK3P, IFIT2, KREMEN1, PDE4D, GBP5, IFITM1, IL27, KCNJ2-AS1, SERPING1, STAT1, TNFRSF21 and VAMP5.

12. The use of the test kit, the kit of parts or the array according to any one of the claims 9 to 11 in a method according to any one of the claims 1 to 8.

13. The *in vitro* use of the genes as defined in claims 1 to 3 in diagnosing or predicting the clinical outcome of the tuberculosis infection, for treatment monitoring and determining treatment duration, and/or determining the therapy-end for the tuberculosis treatment, respectively, by determining the expression level of said genes in a sample of an individual afflicted with or suspected to be afflicted with tuberculosis infection and comparing the level or the corresponding rate or score with a reference value.

14. A computer-implemented method of diagnosing or predicting the clinical outcome of the tuberculosis infection, for treatment monitoring and determining treatment duration, and/or determining the therapy-end for the tuberculosis treatment, respectively, comprising the steps of:

a) obtaining data of measured expression level of the genes as defined in claim 1, 2 and/or 3 from a sample of an individual and, optionally, of controls;
b) computing the data of step a) by comparing the score and/or value and/or said expression level of the genes in said sample with the score and/or value and/or said expression level of the genes obtained from a database or obtained as a measured level of a cut off control;
c) diagnosing or predicting the clinical outcome of the tuberculosis infection, for treatment monitoring and determining treatment duration, and/or determining the therapy-end for the tuberculosis treatment, respectively,
d) optionally further comprising the step of showing the data of diagnosing or predicting the clinical outcome of the tuberculosis infection, for treatment monitoring and determining treatment duration, and/or of determining the therapy-end for the tuberculosis treatment, respectively, on an output unit, in particular showing the results of the diagnosis of tuberculosis infection or the therapy-end for the tuberculosis treatment.

15. Computer readable medium or computer program product having computer executable instructions for performing the steps as identified in any one of the claims1 to 9 or 15.

1A) Therapy End Prediction - German Identification Cohort

Figure 1A

**1B) Therapy End Prediction - German Validation Cohort**

Figure 1B

1C) Therapy End Prediction - Romanian Validation Cohort

Figure 1C

Figure 2

Figure 3 A-D

Figure 3 E-F

Figure 4

22

a)

Figure 5a

23

Figure 5b

Figure 6a

Figure 6b

Europäisches
Patentamt

European
Patent Office

Office européen
des brevets

# EUROPEAN SEARCH REPORT

Application Number

EP 20 15 8652

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/066641 A1 (UNIV LELAND STANFORD JUNIOR [US]) 20 April 2017 (2017-04-20) * p. 2 l. 14 to p. 3 l. 12,p. 4 l. 30-33,p. 26 l. 19-24 and example 1 * | 1,3-15 | INV. C12Q1/6883 |
| X | US 2011/129817 A1 (BANCHEREAU JACQUES F [US] ET AL) 2 June 2011 (2011-06-02) * [0003],[0016] * | 1-15 | |
| X | WO 2014/093872 A1 (BAYLOR RES INST [US]; MEDICAL RES COUNCIL [GB] ET AL.) 19 June 2014 (2014-06-19) * claims 1-9 * | 1,3-15 | |
| X | US 2019/249228 A1 (PENN-NICHOLSON ADAM GARTH [ZA] ET AL) 15 August 2019 (2019-08-15) * [0005] and [0006] (claims 1-3 and 30) * | 1-15 | |
| X | SYLVAIN PRADERVAND ET AL: "Affymetrix Whole-Transcript Human Gene 1.0 ST array is highly concordant with standard 3' expression arrays", BIOTECHNIQUES, vol. 44, no. 6, 1 May 2008 (2008-05-01), pages 759-762, XP055029147, ISSN: 0736-6205, DOI: 10.2144/000112751 * abstract * | 11 | TECHNICAL FIELDS SEARCHED (IPC) C12Q |

The present search report has been drawn up for all claims

| Place of search | Date of completion of the search | Examiner |
|---|---|---|
| Munich | 30 September 2020 | Lapopin, Laurence |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

Application Number

EP 20 15 8652

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

**Europäisches Patentamt**

**European Patent Office**

**Office européen des brevets**

## LACK OF UNITY OF INVENTION
## SHEET B

Application Number

EP 20 15 8652

The Search Division considers that the present European patent application does not comply with the requirements of unity of invention and relates to several inventions or groups of inventions, namely:

1. claims: 1(completely); 3-15(partially)

    relate to a method for diagnosing tuberculosis (TB) and determining therapy-end, using the group of expression markers 1 of claim 1.
    
    ---

2. claims: 2(completely); 3-15(partially)

    relate to a method for monitoring TB treatment using the group of expression markers of claim 2.
    
    ---

## ANNEX TO THE EUROPEAN SEARCH REPORT
## ON EUROPEAN PATENT APPLICATION NO.

EP 20 15 8652

This annex lists the patent family members relating to the patent documents cited in the above-mentioned European search report.
The members are as contained in the European Patent Office EDP file on
The European Patent Office is in no way liable for these particulars which are merely given for the purpose of information.

30-09-2020

| Patent document cited in search report | | Publication date | Patent family member(s) | | Publication date |
|---|---|---|---|---|---|
| WO 2017066641 | A1 | 20-04-2017 | AU | 2016340121 A1 | 24-05-2018 |
| | | | CA | 3001134 A1 | 20-04-2017 |
| | | | CN | 108368551 A | 03-08-2018 |
| | | | EP | 3362579 A1 | 22-08-2018 |
| | | | US | 2018291452 A1 | 11-10-2018 |
| | | | WO | 2017066641 A1 | 20-04-2017 |
| US 2011129817 | A1 | 02-06-2011 | AR | 080570 A1 | 18-04-2012 |
| | | | AU | 2010325179 A1 | 05-07-2012 |
| | | | BR | 112012013029 A2 | 04-10-2016 |
| | | | CA | 2782211 A1 | 03-06-2011 |
| | | | CL | 2012001400 A1 | 09-05-2014 |
| | | | CN | 102844444 A | 26-12-2012 |
| | | | EA | 201270650 A1 | 28-06-2013 |
| | | | EP | 2519652 A2 | 07-11-2012 |
| | | | JP | 2013511981 A | 11-04-2013 |
| | | | KR | 20120107979 A | 04-10-2012 |
| | | | KR | 20140078768 A | 25-06-2014 |
| | | | PE | 20121690 A1 | 16-12-2012 |
| | | | SG | 10201407855W A | 29-01-2015 |
| | | | TW | 201131032 A | 16-09-2011 |
| | | | US | 2011129817 A1 | 02-06-2011 |
| | | | US | 2014080732 A1 | 20-03-2014 |
| | | | WO | 2011066008 A2 | 03-06-2011 |
| | | | ZA | 201204806 B | 27-02-2013 |
| WO 2014093872 | A1 | 19-06-2014 | CA | 2895133 A1 | 19-06-2014 |
| | | | EP | 2931923 A1 | 21-10-2015 |
| | | | US | 2015315643 A1 | 05-11-2015 |
| | | | WO | 2014093872 A1 | 19-06-2014 |
| US 2019249228 | A1 | 15-08-2019 | EP | 3374523 A1 | 19-09-2018 |
| | | | US | 2019249228 A1 | 15-08-2019 |
| | | | WO | 2017081618 A1 | 18-05-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DARBOE F. et al.** *Front Microbiol,* 2019, vol. 10, 1441
- **THOMPSON, E. G. et al.** *Tuberculosis (Edinb),* 2017, vol. 107, 48-58
- *New England Journal of Medicine,* 2016, vol. 375, 11
- **HEYCKENDORF, J. et al.** *Eur Respir J,* 2018, vol. 51
- **HEYCKENDORF, J. et al.** *Int J Tuberc Lung Dis,* 2018, vol. 22, 399-406
- Definitions and reporting framework for tuberculosis - 2013 revision. **GUNTHER, G. et al.** N Engl J Med. World Health Organization, December 2014, vol. 375, 1103-1105

- **SULIMAN, S. et al.** *Am J Respir Crit Care Med,* 2018
- **ZAK, D.E. et al.** *Lancet,* 2016, vol. 387, 2312-2322
- **BLOOM, C.I. et al.** *PLoS ONE,* 2012, vol. 7, e46191-e46191
- **DARBOE, F. et al.** *Front Microbiol,* 2019, vol. 10, 1441
- **THOMPSON, E.G. et al.** *Tuberculosis (Edinb),* 2017, vol. 107, 48-58